# FASCICULE DE BREVET EUROPEEN

(11) **EP 1 443 981 B1**
(45) Date de publication et mention de la délivrance du brevet: **20.05.2015**
(21) Numéro de dépôt: 02760036.0
(22) Date de dépôt: 01.10.2002
(51) Int. Cl.: A61L 24/02, A61K 6/033, A61K 6/06

(54) **MULTICOMPOSANTS PATEUX POUR CIMENTS PHOSPHOCALCIQUES INJECTABLES**
PASTÖSER MEHRKOMPONENTEN-KNOCHENZEMENT
PASTY MULTIPLE CONSTITUENT COMPOSITIONS FOR INJECTABLE CALCIUM PHOSPHATE CEMENTS

(30) Priorité: 14.11.2001 CH 208601
(43) Date de publication de la demande: 11.08.2004
(73) Titulaire: Ecole Polytechnique Fédérale de Lausanne (EPFL), 1015 Lausanne (CH)
(72) Inventeur: LEMAITRE, Jacques, CH-1012 Lausanne (CH); PITTET, Christian, 8050 Zürich (CH); BRENDLEN, David, CH-1024 Ecublens (CH)
(74) Mandataire: Besse, François
(86) Numéro de dépôt international: PCT/CH2002/000543
(87) Numéro de publication internationale: WO 2003/041753

(56) Documents cités:
- WO-A-01/41824
- WO-A-99/17710
- US-A- 4 288 355
- US-A- 5 129 905
- US-A- 5 496 399
- US-A- 5 522 893
- LEMAITRE J: "CIMENTS HYDRAULIQUES PHOSPHO-CALCIQUES: DEVELOPPEMENTS RECENTS ET APPLICATIONS POTENTIELLES", INNOVATION ET TECHNOLOGIE EN BIOLOGIE ET MEDECINE, LILLE, FR, vol. 16, no. 1, suppl, 1 January 1995 (1995-01-01), pages 109-120, XP009094146,

## Description

### Domaine technique

La présente invention concerne les ciments de comblement osseux injectables, plus particulièrement les ciments phosphocalciques hydrauliques.

### Etat de la technique

Les ciments de comblement osseux injectables se présentent sous deux formes bien distinctes : les ciments acryliques durcissant par une réaction de polymérisation et les ciments phosphocalciques hydrauliques (CPHC : Calcium Phosphate Hydraulic Cements) durcissant par réaction de dissolution et précipitation d'espèces contenant des phosphates et/ou des cations calciums.

Les ciments CPHC concernés dans la présente invention durcissent à partir du moment où l'on mélange une ou plusieurs poudres sèches à une certaine quantité d'un composant liquide. Cela implique que les composants de départ sont au moins au nombre de deux et que du moment où ces composants sont mélangés, le temps est compté car la réaction de prise est activée et la pâte ainsi obtenue commence à durcir, jusqu'à former un corps solide.

Le peu de temps à disposition pour le mélange, ainsi que la nécessité d'opérer ce mélange dans des conditions stériles ont poussé les fabricants de ciments de comblement osseux à proposer des systèmes de mélanges mécaniques. Ces systèmes présentent l'avantage d'une meilleure reproductibilité des propriétés du ciment obtenu, ainsi que d'une facilité d'emploi accrue (par rapport à un mélange réalisé à la main). Cependant, les systèmes développés sont souvent compliqués, encombrants et relativement chers. Cette complexité provient principalement du fait que l'obtention d'un mélange homogène à partir d'une poudre sèche et d'un liquide est une opération délicate. Un système de mélange pour ciments phosphocalciques a été proposé dans le brevet EP0976443. Celui-ci permet l'obtention d'une pâte de rapport solide / liquide identique à celui des quantités mélangées (la totalité de la poudre est effectivement mélangée), difficulté que le système du brevet WO98/15314 n'a pas surmontée, selon les inventeurs du brevet EP0976443. Un autre système de mélange, pour le ciment Norian SRS^{®}, a été breveté (US 6149655), mais il prouve bien la difficulté d'obtention d'une pâte homogène à partir d'une poudre et d'un liquide puisque qu'il préconise de répéter le geste de mélange 60 à 90 fois avant l'obtention d'une pâte homogène. Pour les ciments acryliques également, un nombre important de systèmes de mélange a été breveté, mais ils sont aussi compliqués et encombrants.

Les systèmes développés à ce jour pour les ciments de comblement osseux sont donc compliqués, encombrants, relativement chers et présentent une étape de mélange séparée de celle de l'injection.

La présente invention propose notamment un nouveau procédé pour la préparation et l'injection de ciment. Celui-ci permet une diminution des manipulations de la pâte de ciment ainsi qu'un gain de temps entre le moment du mélange et celui de l'injection. Ceci est possible en mélangeant deux composants pâteux qui contiennent chacun une espèce réactive. En effet, le temps nécessaire à l'obtention d'une pâte homogène à partir de deux pâtes est beaucoup plus court qu' à partir d'une poudre et d'un liquide car l'étape de mouillage des poudres est déjà réalisée au moment de mélanger les pâtes. Le présent procédé propose donc de présenter les composants de départ de ciments hydrauliques phosphocalciques sous forme de pâtes.

De plus, en effectuant un dégazage préalable des composants pâteux, le procédé selon l'invention permet l'obtention d'un ciment désaéré de compacité supérieure par rapport aux ciments phosphocalciques de l'état de la technique.

### Exposé de l'invention

La présente invention concerne la présentation d'un ciment phosphocalcique sous forme d'au moins deux composants de départ pâteux, contenant chacun une espèce réactive en suspension. Lesdits composants ne précipitent pas et ne forment donc pas de ciment tant qu'ils ne sont pas mélangés, la réaction de précipitation ne se produisant que lors du mélange desdits composants.

Cette présentation sous forme pâteuse permet par exemple l'utilisation aisée du produit injectable en dentisterie ou en chirurgie, par exemple à l'aide d'une seringue ou d'un pistolet à double corps qui contient un passage commun pour le mélange des composants.

Cette présentation permet au clinicien de s'affranchir de l'étape de mélange manuelle puisqu'elle permet d'opérer en même temps le mélange et l'injection par simple action sur un piston ou une gâchette. De plus, l'affranchissement du mélange manuel classique à la spatule permet d'éviter l'incorporation de bulles d'air dans la pâte, celles-ci étant préjudiciables aux propriétés mécaniques du ciment. Afin de rendre le ciment durci le plus compact possible et d'assurer des propriétés mécaniques les plus reproductibles possibles, les pâtes sont dégazés au moment de leur préparation.

L'avantage du mélange de deux composants pâteux réside dans le fait que le mélange peut se faire pendant l'injection, ce qui limite les étapes, ainsi que les manipulations du ciment.

Il est possible d'appliquer la présente invention à tout type de ciment phosphocalcique en utilisant les réactions de synthèse de ciment pour déterminer les espèces réactives à utiliser. De telles réactions se trouvent en grand nombre dans la littérature et notamment dans la publication : J. Lemaitre « Injectable calcium phosphate hydraulic cements : new developments and potential applications », Inn. Technol. Biol. Med. Vol. 16 (Sp. N°1), 109, 1995.

On peut notamment citer les ciments de type brushitique qui peuvent être utilisés. Ceux-ci sont obtenus à partir de mélanges de phosphate tricalcique et de phosphate monocalcique monohydraté et d'autres sels peu solubles de calcium ou de sodium en milieu aqueux.

Selon la durée de stabilité souhaitée des composants pâteux, l'homme du métier choisira les réactions dont les espèces réactives présentent la stabilité en solution désirée. En effet, l'homme du métier sait calculer la stabilité des substances réactives dans des solutions aqueuses en appliquant, par exemple, les calculs de solubilité des phosphates en milieu aqueux exposés dans la publication : G. Vereecke et J. Lemaitre «Calculation of solubility diagrams in the system Ca(OH)2-H3PO4-KOH-HNO3-CO2-H2O.» Journal of Crystal Growth vol. 104, 820, 1990.

### Modes de réalisation

### EXEMPLE 1 : ciment phosphocalcique de type brushitique présenté sous forme de deux composants de départ pâteux.

La réaction ayant lieu au moment du mélange des deux pâtes est la suivante :

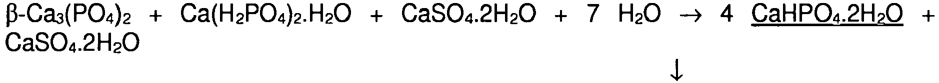

Les pâtes peuvent se présenter de la manière suivante :
- Pâte 1 :
   - 1.142 g de β-Ca₃(PO₄)₂,
   - 0.636 g de solution de Na₂H₂P₂O₇ 50 mmol/l
- Pâte 2 :
   - 0.743 g de Ca(H₂PO₄)₂.H₂O,
   - 0.322 g de CaSO₄.2H₂O,
   - 0.514 g d'eau

Le plâtre CaSO₄.2H₂O entre dans la composition de la pâte 2 pour des raisons annexes (retard de la prise du ciment, meilleure tolérance du ciment in vivo, etc.).

Il est possible d'ajouter à la pâte 1 ou 2, ou dans les deux, des adjuvants polymériques, des stabilisants de suspension, des retardateurs de prise (par exemple Na₂H₂P₂O₇), des radio-opacifiants, par exemple à base d'iode, ou encore des agents médicamenteux (antibiotiques, antimitotiques, agents promoteurs de la repousse osseuse, etc.).

EXEMPLE 2 : ciment phosphocalcique de type de l'exemple 4 du brevet US5522893 (WO9420064) de Chow et Takagi.

La réaction ayant lieu au moment du mélange des deux pâtes est la suivante :

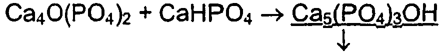

Les pâtes peuvent se présenter de la manière suivante :
- Pâte 1 :
   - 3.66 g de Ca₄O(PO₄)₂,
   - 1.85 g d'eau stérile
- Pâte 2 :
   - 1.36 g de CaHPO₄,
   - 2.30 g de Ca₅(PO₄)₃OH,
   - 1.85 g d'acide orthophosphorique 10 mmol/l

EXEMPLE 3 : Ciment phosphocalcique apatitique de type Norian SRS^{®}. Ce type de ciment est décrit dans le brevet US 5129905.

La réaction ayant lieu au moment du mélange des deux pâtes est la suivante :

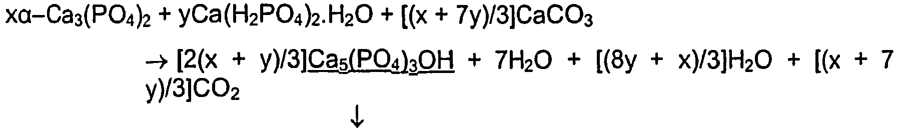

Les pâtes peuvent se présenter de la manière suivante :
- Pâte 1 :
   - 1.53 g de α-Ca₃(PO₄)₂,
   - 0.31 g de CaCO₃,
   - 0.90 g de Na₂HPO₄ 100 mmol/l
- Pâte 2 :
   - 0.16 g de Ca(H₂PO₄)₂.H₂O,
   - 1.68 g de Ca₅(PO₄)₃OH,
   - 0.80 g d'acide orthophosphorique 10 mmol/l

Les ciments obtenus selon la méthode de l'invention sont plus denses que ceux obtenus par les méthodes traditionnelles impliquant une ou plusieurs poudres sèches mélangées à un composant liquide. Ceci est le résultat du dégazage des pâtes de départ, étape qui n'est pas possible dans les méthodes connues car on ne dispose pas du temps nécessaire.

A l'instar de l'exemple 1, il est possible d'ajouter aux pâtes des exemples 2 et 3 différentes substances.

## Revendications

1. Procédé de fabrication d'un ciment phosphocalcique destiné à être injecté, qui est obtenu par mélange d'au moins deux composants de départ, **caractérisé par le fait que** chaque composant de départ :
- est sous forme de pâte,
- contient au moins une espèce réactive en suspension,
- lesdits composants ne précipitant pas et ne formant pas de ciment tant qu'ils ne sont pas mélangés,
et qu'une étape de dégazage est effectuée sur lesdits composants avant de les mélanger.

2. Procédé selon la revendication 1, **caractérisé en ce que** le ciment phosphocalcique est du DCPD (dicalcium phosphate dihydrate) et les espèces réactives du β-TTCP (beta tricalcium phosphate) et du MCPM (monocalcium phosphate monohydrate).

3. Procédé selon la revendication 1, **caractérisé en ce que** le ciment phosphocalcique est de l'OHAP (hydroxyapatite) et les espèces réactives du TTCP (tetracalcium phosphate monoxide) et du DCPA (dicalcium phosphate anhydre).

4. Procédé selon la revendication 1, **caractérisé en ce que** le ciment phosphocalcique est de l'OHAP (hydroxyapatite) et les espèces réactives de l'α-TCP (alpha tricalcium phosphate), du MCPM (monocalcium phosphate monohydrate) et du CC (carbonate de calcium).

5. Procédé selon l'une quelconque des revendications précédentes, **caractérisé en ce que** les composants pâteux comprennent de l'eau.

6. Procédé selon l'une quelconque des revendications précédentes, **caractérisé en ce que** des adjuvants polymériques et/ou des stabilisants de suspension et/ou des retardateurs de prise et/ou des radio-opacifiants et/ou encore des agents médicamenteux sont ajoutés à un ou à plusieurs desdits composants pâteux.

7. Ensemble constitué d'au moins deux composants pâteux tels que définis dans l'une quelconque des revendications 1 à 6 et d'une seringue à double corps contenant un passage commun pour le mélange desdits composants, chaque composant comprenant une ou plusieurs espèces réactives qui, en réagissant avec la ou les espèce(s) réactive(s) du ou des autres composants, résulte en l'obtention d'un ciment phosphocalcique.

8. Utilisation des composants selon la revendication 1, **caractérisée par le fait que** lesdits composants sont introduits dans un objet à plusieurs compartiments, un système de mélange étant prévu pour la mise en contact desdits composants pendant l'injection.

9. Composants pâteux tels que définis dans l'une quelconque des revendications 1 à 6 pour leur mise en oeuvre en chirurgie ou dentisterie.

## Patentansprüche

1. Verfahren zur Herstellung eines Calciumphosphat-Zements zur Injektion, der erhalten wird durch Mischen von mindestens zwei Ausgangsbestandteilen, **dadurch gekennzeichnet, dass** jeder Ausgangsbestandteil:
- in Pastenform vorliegt,
- mindestens eine suspendierte reaktive Spezies enthält,
- die Bestandteile nicht ausfallen und erst einen Zement bilden, wenn sie gemischt werden,
und dadurch, dass ein Entgasungsschritt an den Bestandteilen durchgeführt wird, bevor sie gemischt werden.

2. Verfahren nach Anspruch 1, **dadurch gekennzeichnet, dass** es sich bei dem Calciumphosphat-Zement um DCPD (Dicalciumphosphatdihydrat) und bei den reaktiven Spezies um β-TCP (beta-Tricalciumphosphat) und MCPM (Monocalciumphosphatmonohydrat) handelt.

3. Verfahren nach Anspruch 1, **dadurch gekennzeichnet, dass** es sich bei dem Calciumphosphat-Zement um OHAP (Hydroxyapatit) und bei den reaktiven Spezies um TTCP (Tetracalciumphosphatmonoxid) und DCPA (wasserfreies Dicalciumphosphat) handelt.

4. Verfahren nach Anspruch 1, **dadurch gekennzeichnet, dass** es sich bei dem Calciumphosphat-Zement um OHAP (Hydroxyapatit) und bei den reaktiven Spezies um α-TCP (alpha-Tricalciumphosphat), MCPM (Monocalciumphosphatmonohydrat) und CC (Calciumcarbonat) handelt.

5. Verfahren nach einem der vorhergehenden Ansprüche, **dadurch gekennzeichnet, dass** die pastenförmigen Bestandteile Wasser umfassen.

6. Verfahren nach einem der vorhergehenden Ansprüche, **dadurch gekennzeichnet, dass** polymere Hilfsstoffe und/oder Suspensionsstabilisatoren und/oder Abbindeverzögerer und/oder röntgendichte Mittel und/oder Arzneimittel zu einem oder mehreren dieser pastenförmigen Bestandteile hinzugegeben werden.

7. Kit, bestehend aus mindestens zwei pastenförmigen Bestandteilen nach einem der Ansprüche 1 bis 6 und einer Doppelzylinderspritze, die einen gemeinsamen Kanal zum Mischen der Bestandteile enthält, wobei jeder Bestandteil eine oder mehrere reaktive Spezies umfasst, die bei der Reaktion mit der oder den reaktiven Spezies des oder der anderen Bestandteil(s/e) zum Erhalt eines Calciumphosphat-Zements führt/führen.

8. Verwendung von Bestandteilen nach Anspruch 1, **dadurch gekennzeichnet, dass** die Bestandteile in einen Gegenstand mit mehreren Kompartimenten eingebracht werden, wobei ein Mischsystem zum In-Kontakt-Bringen der Bestandteile während der Injektion vorgesehen ist.

9. Pastenförmige Bestandteile nach einem der Ansprüche 1 bis 6 für die Verwendung in der Chirurgie oder der Zahnmedizin.

## Claims

1. Process for the manufacture of a calcium phosphate cement intended to be injected, which is obtained by mixing at least two starting constituents, **characterized in that** each starting constituent:
- is in the paste form,
- comprises at least one reactive entity in suspension,
- said constituents not precipitating and not
forming a cement as long as they are not mixed, and **in that** a stage of degassing is carried out on said constituents before they are mixed.

2. Process according to Claim 1, **characterized in that** the calcium phosphate cement is DCPD (dicalcium phosphate dihydrate) and the reactive entities β-TTCP (β-tricalcium phosphate) and MCPM (monocalcium phosphate monohydrate).

3. Process according to Claim 1, **characterized in that** the calcium phosphate cement is OHAP (hydroxyapatite) and the reactive entities TTCP (tetracalcium phosphate monoxide) and DCPA (anhydrous dicalcium phosphate).

4. Process according to Claim 1, **characterized in that** the calcium phosphate cement is OHAP (hydroxyapatite) and the reactive entities α-TCP (α-tricalcium phosphate), MCPM (monocalcium phosphate monohydrate) and CC (calcium carbonate).

5. Process according to any one of the preceding claims, **characterized in that** the pasty constituents comprise water.

6. Process according to any one of the preceding claims, **characterized in that** polymeric adjuvants and/or suspension stabilizers and/or setting retardants and/or radiopaques and/or medicines are added to one or to several of said pasty constituents.

7. Combination composed of at least two pasty constituents as defined in any one of Claims 1 to 6 and of a twin-chambered syringe comprising a common passage for the mixing of said constituents, each constituent comprising one or more reactive entities which, by reacting with the reactive entity(ies) of the other constituent or constituents, results in the production of a calcium phosphate cement.

8. Use of the constituents according to Claim 1, **characterized in that** said constituents are introduced into a multicompartment device, a mixing system being provided for bringing said constituents into contact during the injection.

9. Pasty constituents as defined in any one of Claims 1 to 6, for their use in surgery or dentistry.
